Europäisches Patentamt

European Patent Office

Office européen des brevets

(11) Veröffentlichungsnummer: **0 032 164**
**B1**

(12) # EUROPÄISCHE PATENTSCHRIFT

(45) Veröffentlichungstag der Patentschrift:
28.09.83

(51) Int. Cl.³: **A 61 F 1/03**

(21) Anmeldenummer: **80106673.9**

(22) Anmeldetag: **30.10.80**

(54) **Blattartiger Schaft für eine Gelenkendoprothese.**

(30) Priorität: **14.01.80 CH 256/80**

(43) Veröffentlichungstag der Anmeldung:
**22.07.81 Patentblatt 81/29**

(45) Bekanntmachung des Hinweises auf die Patenterteilung:
**28.09.83 Patentblatt 83/39**

(84) Benannte Vertragsstaaten:
**AT DE FR GB IT**

(56) Entgegenhaltungen:
**DE-A-2 246 940**
**DE-B-1 541 246**
**US-A-3 740 769**
**US-A-3 765 034**
**US-A-3 808 606**
**US-A-3 815 590**

(73) Patentinhaber: **GEBRÜDER SULZER AKTIENGESELLSCHAFT**, Zürcherstrasse 9, **CH-8401 Winterthur (CH)**

(72) Erfinder: **Zweymüller, Karl, Dr.-med., Döblinger Hauptstrasse 22/5, A-1190 Wien (AT)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

## Blattartiger Schaft für eine Gelenkendoprothese

Die Erfindung betrifft einen blattartigen Schaft einer Gelenkendoprothese, insbesondere einer Hüftgelenkprothese, wobei der Schaft sich vom distalen Ende aus zunächst konisch erweitert und seine Längsmittelachse mit der Achse eines den Gelenkteil tragenden Zapfens einen Winkel bildet, und wobei auf dem Schaftblatt mindestens drei Markierungspunkte festgelegt sind.

Ein Vergleich von zu verschiedenen Seiten aufgenommenen Röntgenbildern implantierter Gelenkendoprothesen bereitet oft Schwierigkeiten; derartige Vergleiche sind jedoch notwendig, um Verschiebungen, beispielsweise ein Einsinken oder Setzen der Implantate im Knochen möglichst frühzeitig feststellen zu können. Die Schwierigkeiten basieren zum einen darauf, daß z. B. verschiedene Abbildungsmaßstäbe bei den verschiedenen Röntgenbildern zustande gekommen sind. Zum anderen können nicht vorhandene Änderungen im Sitz der Prothese vorgetäuscht werden, wenn die Lage der durch das ersetzte Gelenk verbundenen Glieder relativ zur Aufnahmerichtung bei den verschiedenen Aufnahmen nicht die gleiche ist.

Aus der DE-A-2 246 940 ist eine Prothese mit einem blattartigen Schaft der anmeldungsgemäßen Gattung bekannt, der eine Reihe von in einer Linie hintereinander liegenden Bohrungen als Markierungspunkte hat; sie dienen dazu, im Zusammenwirken mit einem durch die Bohrungen gesteckten Stift die richtige Tiefe für das Einsetzen des Schaftes in den Femurknochen während der Implantationsoperation zu ermitteln. Da die Markierungspunkte in einer Linie angeordnet sind, lassen sich Änderungen im Abbildungsmaßstab und in der relativen Lage des Schaftblattes zur Aufnahmeebene bei zu verschiedenen Zeiten hergestellten Röntgenaufnahmen nicht ermitteln.

Aufgabe der Erfindung ist es, den Vergleich von zu verschiedenen Zeiten hergestellten Röntgenaufnahmen zu erleichtern. Erfindungsgemäß wird diese Aufgabe dadurch gelöst, daß die Markierungspunkte des Schaftblattes als Röntgenmarkierungspunkte eine Dreiecksfläche einschließen, wobei mindestens je einer der Punkte auf der Längsmittelachse und auf der Zapfenachse angeordnet ist, und daß ferner die Abstände dieser Punkte auf dem Schaftblatt vermessen sind.

Die eine — möglichst große — Fläche einschließenden Markierungspunkte, deren tatsächliche Abstände genau vermessen und bekannt sind, ermöglichen, durch Ausmessen ihrer Bildpunkte auf den Aufnahmen Änderungen des Abbildungsmaßstabes und/oder des Winkels, den die Oberfläche des Schaftblattes mit der Aufnahmerichtung bildet, auf einfache Weise festzustellen. Sind darüber hinaus bei einer beispielsweise unmittelbar nach der Implantation gemachten Aufnahme die Abstände der Markierungspunkte zu markanten Stellen des Knochens — beispielsweise zur Oberkante des großen Trochanters oder zur Spitze des kleinen Trochanters bei einer Hüftgelenkprothese — vermessen worden, so lassen sich nach Ausmessen dieser Abstände in späteren Aufnahmen Lageänderungen der Prothesen im Knochen ebenfalls ohne weiteres ermitteln.

Besonders vorteilhaft ist es, wenn die Markierungspunkte durch die Mittelpunkte von Bohrungen durch das Schaftblatt gegeben sind; denn durch die elliptischen Verzerrungen, die die Bohrungen erfahren, wenn z. B. die Aufnahmerichtung nicht senkrecht zur Oberfläche des Schaftblattes ist, läßt sich mindestens näherungsweise schon ohne Vermessen der Bildpunkte eine falsche oder zumindest eine geänderte Winkellage zwischen dem Schaftblatt und der Aufnahmerichtung erkennen.

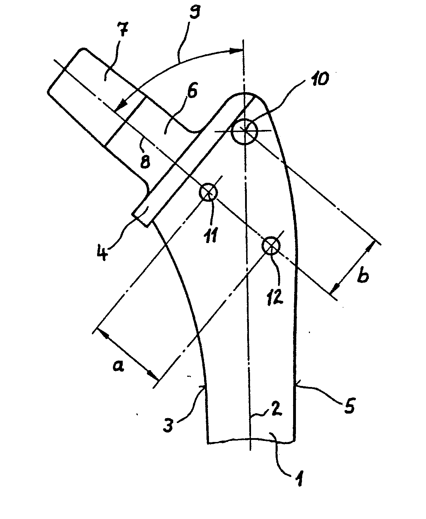

Im folgenden wird die Erfindung anhand eines Ausführungsbeispiels im Zusammenhang mit der Zeichnung näher erläutert, deren einzige Figur schematisch den oberen Teil eines Schaftes für eine Hüftgelenkprothese in einer Ansicht auf die Oberfläche des Schaftblattes zeigt.

Das Schaftblatt 1 verläuft in seinem nicht dargestellten unteren Teil sich konisch nach oben erweiternd, wobei im gezeigten Beispiel der Konus symmetrisch zu einer Längsmittelachse 2 ausgebildet ist. Die mediale Schmalseite 3 des Konus geht in einen Bogen über, der in einem Kragen 4 endet. Ebenso führt die laterale Schmalseite 5 in einem Bogen zum Kragen 4.

Auf der anderen Seite des Kragens 4 ist über einen Hals 6 ein sich nach außen konisch verjüngender Zapfen 7 aufgesetzt, der den nicht gezeigten kugelförmigen Gelenkkopf aufnimmt. Die Achse 8 des Zapfens 7 schneidet die Längsmittelachse 2 des Schaftes unter einem Winkel 9, der im wesentlichen den Winkel zwischen dem Schenkelhals und der Femurachse eines natürlichen Hüftgelenkes entspricht und im vorliegenden Fall 49° beträgt.

Erfindungsgemäß führen durch das Schaftblatt 1 als Markierungspunkte 10 – 12 drei Bohrungen, deren Mittelpunkte eine Dreiecksfläche einschließen. Der Mittelpunkt der im Durchmesser gegenüber den beiden anderen 11 und 12 etwas größeren Bohrung 10 liegt dabei auf der Längsmittelachse 2 des Schaftblattes 1, während die Bohrungen 11 und 12 auf der Zapfenachse 8 in einem genau vermessenen Abstand a hintereinander angeordnet sind.

Um Größe und Lage der von den Bohrungen 10 – 12 eingeschlossenen Dreiecksfläche festzulegen, ist darüber hinaus zusätzlich mindestens noch der senkrechte Abstand b, den die Bohrung 10 von der Achse 8 hat, ausgemessen.

Die Abstände a und b können an sich willkürlich gewählt werden; es ist jedoch zweckmäßig, sie so groß wie — ohne Schwächung der mechanischen Eigenschaften des Schaftes, beispielsweise seiner Festigkeit — möglich zu wählen, da dadurch die relative Genauigkeit für ihre Meßwerte auf den Bildern verbessert wird. Weder in der Lage der Markierungspunkte 10—12 auf den beiden genannten Achsen 2 und 8 noch in der Realisierung als Mittelpunkte der Bohrungen ist die Erfindung auf das gezeigte Ausführungsbeispiel beschränkt. So können die Markierungspunkte auch beispielsweise als Vorsprünge, einfache Vertiefungen oder kerbenartige Einschnitte verwirklicht sein.

## Patentansprüche

1. Blattartiger Schaft einer Gelenkendoprothese, insbesondere einer Hüftgelenkprothese, wobei der Schaft (1) sich vom distalen Ende aus zunächst konisch erweitert und seine Längsmittelachse (2) mit der Achse (8) eines den Gelenkteil tragenden Zapfens (7) einen Winkel (9) bildet, und wobei auf dem Schaftblatt (1) mindestens drei Markierungspunkte (10—12) festgelegt sind, dadurch gekennzeichnet, daß die Markierungspunkte (10—12) des Schaftblattes (1) als Röntgenmarkierungspunkte eine Dreiecksfläche einschließen, wobei mindestens je einer der Punkte (10—12) auf der Längsmittelachse (2) und auf der Zapfenachse (8) angeordnet ist, und daß ferner die Abstände (a, b) dieser Punkte (10—12) auf dem Schaftblatt (1) vermessen sind.

2. Schaft nach Anspruch 1, dadurch gekennzeichnet, daß die Markierungspunkte (10—12) durch die Mittelpunkte von Bohrungen durch das Schaftblatt (1) gegeben sind.

## Claims

1. A blade-like stem of an articulation endoprosthesis, more particularly a hip joint prosthesis, the stem (1) first widening conically from the distal end, the stem longitudinal central axis (2) making an angle (9) with the axis (8) of a pin (7) carrying the joint part, at least three marking points (10—12) being located on the blade (1), characterised in that the marking points (10—12) of the blade (1) enclose as X-ray marking points a triangular surface, at least one each of the points (10—12) being disposed on the longitudinal central axis (2) and on the pin axis (8), and the distances (a, b) of these points (10—12) on the blade (1) are surveyed.

2. A stem according to claim 1, characterised in that the marking points (10—12) are the central points of bores through the stem blade (1).

## Revendications

1. Tige lamelliforme d'une endoprothèse d'articulation, en particulier une prothèse de la hanche, la tige (1) s'élargissant alors tout d'abord coniquement à partir de l'extrémité éloignée et son centre longitudinal (2) formant un angle (9) avec l'axe (8) d'un tourillon (7) portant la partie d'articulation, et au moins trois points de marquage (10—12) étant déterminés sur la lame de tige (1), caractérisée en ce que les points de marquage (10—12) de la lame de tige (1) décrivent en tant que points de marquage de radiographie une surface triangulaire, au moins un des points respectifs (10—12) étant disposé sur le centre longitudinal (2) et sur l'axe du tourillon (8), et en ce que de plus les distances (a, b) entre ces points (10—12) sont mesurées sur la lame de tige (1).

2. Tige selon la revendication 1, caractérisée en ce que les points de marquage (10—12) sont donnés par les centres de trous formés à travers la lame de tige (1).